(19) Europäisches Patentamt
European Patent Office
Office européen des brevets

(11) Veröffentlichungsnummer: **0 491 211 A1**

## EUROPÄISCHE PATENTANMELDUNG

(21) Anmeldenummer: **91120646.4**

(51) Int. Cl.⁵: **A61B 17/58**

(22) Anmeldetag: **30.11.91**

(30) Priorität: **19.12.90 DE 9017101 U**

(43) Veröffentlichungstag der Anmeldung:
**24.06.92 Patentblatt 92/26**

(84) Benannte Vertragsstaaten:
**AT BE CH DE FR GB LI**

(71) Anmelder: **Synthes AG Chur**
**Grabenstrasse 15**
**CH-7002 Chur(CH)**

(72) Erfinder: **Frigg, Robert**
**Scaletta-Strasse 8**
**CH-7270 Davos-Platz(CH)**

Erfinder: **Perren, Stephan**
**Dischmastrasse 22**
**CH-7260 Davos-Dorf(CH)**
Erfinder: **Allgöwer, Martin, c/o Soc. Int. de**
**Chirurgie**
**Hauptstrasse 63**
**CH-4153 Reinach(CH)**
Erfinder: **Gisin, Paul**
**Brestenbergweg 7**
**CH-4437 Waldenburg(CH)**

(74) Vertreter: **Lusuardi, Werther Giovanni, Dr.**
**Dr. Lusuardi AG, Kreuzbühlstrasse 8**
**CH-8008 Zürich(CH)**

(54) **Knochenschraube.**

(57) Die Knochenschraube (1) weist einen Schraubenkopf (2), eine Schraubenspitze (11) und einen mit einem Gewinde (8,14) versehenen Schaft (5) auf.
Der mit einem Gewinde versehene Abschnitt (6,7) des Schaftes (5) verfügt über einen kopfseitigen, im wesentlichen zylindrischen, ersten Schaftabschnitt (6) mit dem Kerndurchmesser $d_1$ (16) und über einen spitzenseitigen, an den ersten Schaftabschnitt (6) anschliessenden, zweiten Schaftabschnitt (7) mit einem kleineren Kerndurchmesser $d_2$ (17).
Das Gewinde (8,14) in den beiden Schaftabschnitten (6,7) weist die gleiche Steigung (10,12) auf.

Fig.1

EP 0 491 211 A1

Die Erfindung bezieht sich auf eine Knochenschraube gemäss dem Oberbegriff des Anspruchs 1.

Knochenschrauben der genannten Gattung haben grundsätzlich zwei Indikationen.

Die erste Indikation betrifft die Halterung zweier Knochenfragmente in einer vorbestimmten anatomischen Lage zueinander, im speziellen bei der Verkürzungs- und Verlängerungsosteotomie. Bei dieser ersten Indikation wird die Knochenschraube allein verwendet.

Die zweite Indikation betrifft die Verankerung von externen Fixationselementen im Knochen, z.B. bei der Anwendung eines Fixateur externe in der Osteosynthese. Bei dieser zweiten Indikation werden die Knochenschrauben, in Form einer Schanzschen Schraube, innerhalb eines Rahmens aus einem oder mehreren Längsträgern verwendet.

Bei der ersten Indikation, beispielsweise bei unterentwickeltem Untergesicht eines Patienten wird, z.B. durch Spaltung und Fragmentverschiebung des Unterkiefers eine operative Verlängerung des Unterkiefers angestrebt, die einen normalen Zahnüberbiss gestattet. Hierfür wird ein Okklusionsschlüssel als Führungs- und Positionsschiene für die Einstellung des Corpus- und Frontsegmentes des Kiefers eingesetzt. Die Fragmente werden dann mit jeweils mehreren, vorzugsweise drei Zugschrauben fixiert.

Bei der Verwendung üblicher Knochenschrauben treten Zugkräfte auf, die ein genaues Einhalten der operativ vorbestimmten anatomischen Lage der Knochenstücke zueinander verhindern. Gleichzeitig ist z.B. bei der Korrektur der Unterkieferfeststellung nur der Einsatz von zwei haltenden Zugschrauben auf der Zugseite der Linea obliqua möglich, wobei eine, die relative Lage der Bruchstücke zueinander besser definierende und gleichzeitig haltende dritte Knochenschraube nur marginal applizierbar ist. Dadurch ist z.B. bei der Unterkieferosteotomie eine stabile Lage der Fragmentstücke zueinander nicht gewährleistet. Dieselben Nachteile bekannter Knochenschrauben treten natürlich auch bei anderen Indikationen auf, bei denen zwei Knochenfragmente zumeist ohne direkten körperlichen Kontakt zueinander fixiert werden müssen.

Bei der zweiten Indikation wird die erfindungsgemäss Knochenschraube in Form einer Schanzschen Schraube verwendet, welche der Verankerung der externen Fixationselemente im Knochen dienen. Die Schanzschen Schrauben werden durch kleine Stichinzisionen von aussen durch die Haut und durch die Weichteile in den Knochen eingebracht. Das Gewinde der Schanzschen Schrauben verhindert das axiale Verschieben derselben in der Knochenbohrung. Am normalerweise glatten Schaft der Schanzschen Schraube wird das eigentliche Fixationselement, der Fixateur externe, montiert.

Das Hauptgebiet dieser zweiten Indikation ist dasjenige der offenen Frakturen. Diese können nicht mit einer internen Osteosynthese versorgt werden, da die Implantation den Weichteilen zusätzlichen Schaden zufügen würde. Ein Nachteil dieser bekannten Methode ist allerdings die permanente Verbindung des Knochens mit der "Aussenwelt", durch die transkutan applizierten Schanzschen Schrauben. Je länger diese Schanzschen Schrauben im Körper belassen werden, um so höher ist die Gefahr einer sogenannten "Pintrak-Infektion". Eine solche Infektion wird durch die, nicht unter Spannung stehenden Schanzschen Schrauben noch verstärkt, da sie durch Mikrobewegungen eine Knochenresorption hervorrufen. Diese Lockerungen können im Extremfall zu einer Sequesterbildung führen. Klinische Untersuchungen haben gezeigt, dass eine "Pintrak-Infektion" bei guter Verankerung der Schraube im Knochen, sehr selten wäre.

Aus der EP-A2 0 369 266 ist bereits eine Knochenschraube ähnlicher Bauart bekannt, welche zwischen ihrem glatten Schaftteil und ihrem Gewindeteil eine Schulter mit entsprechendem Durchmesseranstieg aufweist, um bei der Implantation im Röhrenknochen eine radiale Vorspannung erzeugen zu können. Nachteilig bei dieser bekannten Knochenschraube ist die Einführung des im Durchmesser erweiterten Schraubenschaftes in die nahe Kortikalis des Röhrenknochens. Wenn das endseitig angebrachte Gewinde in der Gegenkortikalis nicht genügend Halt findet, reicht die Verankerung nicht aus um den konisch sich erweiternden Schraubenschaft in die nahe Kortikalis zu ziehen. Das Gewinde wird in einem solchen Falle ausgerissen und es ist nicht mehr möglich, die Schraube durch axialen Druck in den Knochen einzudrehen. Ein weiteres Problem bei dieser bekannten Schraube liegt in der Einhaltung der Bohrdurchmessertoleranz beim Bohren der nahen Kortikalis. Wenn der Bohrer nicht genügend geführt wird, entsteht eine zu grosse Bohrung, welche nicht genügend radial vorgespannt werden kann.

Hier will die Erfindung Abhilfe schaffen. Der Erfindung liegt die Aufgabe zugrunde, eine Knochenschraube der eingangs erwähnten Art zu schaffen, bei der für eine erste Indikation die Fixierung zweier Knochenstücke in einer anatomisch vorbestimmten Lage über die Dauer des Heilungsprozesses sicher gewährleistet ist und bei der für eine zweiten Indikation mit nur einem Bohrvorgang unter einer genau kontrollierten radialen Kompression des aufnehmenden Knochengewebes die Knochenschraube fest in eine unterdimensionierte Bohrung, ohne eine Knochenverletzung zu erzeugen, eingeschraubt werden kann, wobei auch unter zusätzlichen, funktionellen Belastungen der radiale Pressitz über den gesamten Umfang beibehalten wird.

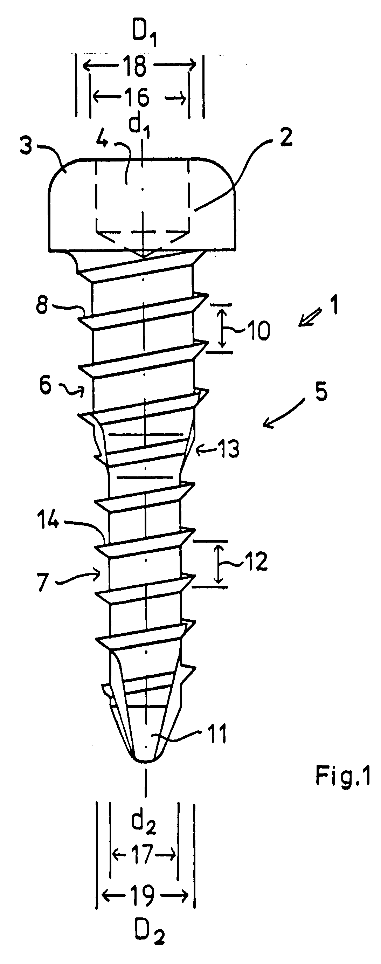

Bei einer bevorzugten Ausführungsform für die erste Indikation (gemäss den Fig. 1 - 3) ist der Aussendurchmesser der Rippen des Aussengewindes des zweiten, vorderen Schaftabschnittes kleiner als der Kerndurchmesser des ersten, hinteren Schaftabschnittes.

Dadurch, dass die Knochenschraube zwei mit einem Gewinde versehene Abschnitte aufweist, welche einen unterschiedlichen Kerndurchmesser aufweisen, ist die Schraube in zwei Fragmentstücken verankerbar, die eine Öffnung mit an den jeweiligen Schaftbereich angepassten Durchmesser aufweisen. Der beim Einschrauben vorbestimmte, anatomische Abstand der beiden Fragmente voneinander bleibt beim Einschrauben der Knochenschraube erhalten. Somit wird ein sich zwischen den Knochenstücken bestehender knochenfreier Hohlraum sicher überbrückt. Der Übergangsbereich ist bei dieser Ausführungsform vorzugsweise kürzer oder gleich einem Gewindegang des Gewindes, welches zweckmässigerweise selbstschneidend ausgebildet ist, so dass der Operateur die Knochenschraube nach dem Setzen der notwendigen Bohrung direkt einsetzen kann.

Vorteilhafterweise ist zwischen den beiden, ein Gewinde aufweisenden Schaftabschnitten ein Übergangsbereich angeordnet, der sich vorzugsweise stetig verjüngt, z.B. in Form eines Konus.

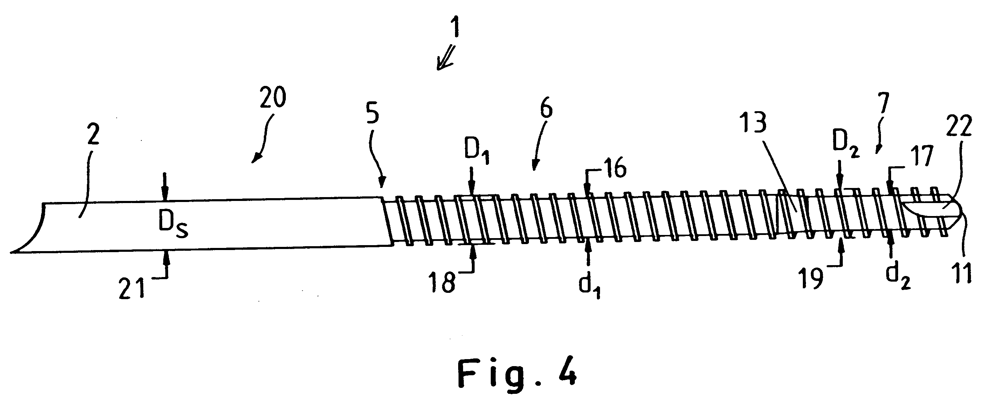

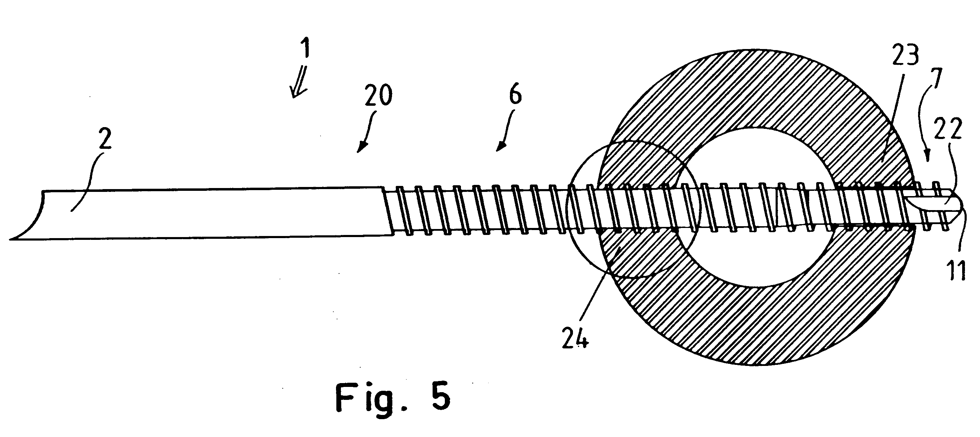

Bei einer weiteren bevorzugten Ausführungsform für die zweite Indikation (gemäss den Fig. 4/5) verfügt der Schaft über einen zusätzlichen, kopfseitig an seinem mit einem Gewinde versehenen Abschnitt anschliessenden, glatten, dritten Schaftabschnitt. Bei dieser Ausführungsform beträgt die Länge des Übergangsbereiches vorteilhafterweise 3 bis 7 mm, vorzugsweise 4 bis 6 mm. Da die Bruchdehnung des Knochens bei etwa 2 - 3 % liegt, sollten die Dimensionen des Übergangsbereiches bei dieser Ausführungsform derart gewählt werden, dass das Verhältnis $d_1$-$d_2$/$d_2$ zwischen 0,004 und 0,020, vorzugsweise zwischen 0,008 und 0,012 liegt. Damit kann eine optimale Pressung des Knochenmaterials erreicht werden.

Zweckmässigerweise besitzt der zweite, spitzenseitige Schaftabschnitt einen Gewindeaussendurchmesser $D_2$, der dem Durchmesser $D_s$ des dritten, kopfseitigen Schaftabschnittes entspricht. Im weiteren ist die Schraubenspitze vorzugsweise selbstschneidend ausgebildet, z.B. in Form eines Trokars oder einer oder mehrerer radial über den Umfang verteilten Schneidnuten. Das Gewinde der Knochenschraube ist zweckmässigerweise derart dimensioniert, dass das Verhältnis zwischen Kerndurchmesser $d_2$ und Aussendurchmesser $D_2$ des zweiten Schaftabschnittes im Bereich zwischen 0,89 und 0,95, vorzugsweise zwischen 0,91 und 0,93 liegt.

Um zu verhindern, dass gleichzeitig der spitzenseitige Abschnitt des Gewindeteils in der hinteren Kortikalis und das Übergangsteil in der vorderen Kortikalis zur Wirkung kommen, wodurch man die Kontrolle beim Eindrehen der Knochenschraube verlieren würde, ist es vorteilhaft den Übergangsbereich in einem Abstand von 15 - 25 mm, vorzugsweise von 18 - 22 mm von der Schraubenspitze anzubringen; seine Länge hängt von den absoluten Dimensionen der Knochenschraube ab und liegt typischerweise zwischen 3 und 7 mm, vorzugsweise zwischen 4 und 6 mm.

Die Länge des ersten Schaftabschnitt des Schaftes hängt ebenfalls von den absoluten Dimensionen der Knochenschraube ab und liegt typischerweise zwischen 60 bis 80 mm, vorzugsweise zwischen 65 bis 75 mm. Die Steigung des Gewindeganges des Gewindes des zweiten Schaftabschnittes liegt vorteilhafterweise im Bereich von 1,5 bis 2 mm, vorzugsweise zwischen 1,7 bis 1,8 mm.

Die durch die Ausführungsform gemäss den Fig. 4 und 5 erreichten Vorteile sind im wesentlichen darin zu sehen, dass dank der geringfügig unterschiedlichen Dimensionierung des Kerndurchmessers des mit einem Gewinde versehenen Schaftabschnittes eine erwünschte radiale Vorspannung in der Knochenbohrung erzeugt wird, welche auch bei zusätzlichen, funktionellen Belastungen der erfindungsgemässen Knochenschraube mindestens teilweise aufrechterhalten bleibt, so dass keine Lockerungen und dadurch bedingte Resorptionen des Knochengewebes auftreten; im weiteren muss nur noch ein einziger gängiger Bohrer verwendet werden um den zweistufigen Kern des Gewindeteils der erfindungsgemässen Knochenschraube fest mit einer radialen Vorspannung, aber ohne Knochenverletzung in die Knochenbohrung eindrehen zu können. Da die radiale Vorspannung bei der erfindungsgemässen Knochenschraube in Form eines überdimensionierten Gewindes durchgeführt wird ist deren Höhe über den Kerndurchmesser des Gewindes genau definiert.

Nachfolgend werden Ausführungsbeispiele der Erfindung anhand der Zeichnung näher erläutert.

Es zeigen:

Fig. 1 eine Seitenansicht einer Knochenschraube;

Fig. 2 eine Unteransicht auf die Knochenschraube nach Fig. 1;

Fig. 3 eine Draufsicht auf die Knochenschraube nach Fig. 1;

Fig. 4 einen Längsschnitt durch bevorzugte Ausführungsform der erfindungsgemässe Knochenschraube; und

Fig. 5 einen Längsschnitt durch die in einem Röhrenknochen implantierte Knochenschraube gemäss Fig. 4.

Die Fig. 1 zeigt schematisch in einer Seitenansicht eine Knochenschraube 1 zur Fixierung von zwei Knochenfragmenten. Dabei kann die Knochenschraube 1 insbesondere bei der operativen Verlängerung oder Verkürzung z.B. des Unterkiefers oder bei der Verlängerung von Gliedmassen eingesetzt werden. Die Knochenschraube 1 verfügt über einen Schraubenkopf 2, der in einer vorteilhaften Ausgestaltung abgerundete Ecken 3 aufweist. Ferner ist in dem Schraubenkopf 2 ein Innensechskant 4 vorgesehen, in den der operierende Arzt ein Werkzeug zur leichten Fixierung der Knochenschraube 1 einsetzen kann.

Die Knochenschraube 1 verfügt über einen Schaft 5, der zwei Schaftabschnitte 6 und 7 aufweist. Der direkt an den Schraubenkopf 2 anschliessende erste Schaftabschnitt 6 ist im wesentlichen kreiszylindrisch mit einem konstanten Kerndurchmesser $d_1$ 16 ausgestaltet. Der Kerndurchmesser $d_1$ 16 des ersten Schaftabschnittes 6 beträgt bei der für eine Kieferosteotomie vorgesehenen Knochenschraube 1 z.B. 9 Millimeter. Der Schaftabschnitt 6 verfügt über ein Aussengewinde 8 mit gleichmässigem Gewindegang 10. Das in der Fig. 1 mit drei Windungen dargestellte Aussengewinde 8 des Schaftabschnittes 6 kann bei Knochenschrauben 1 für andere Anwendungen auch ein Vielzahl von Windungen umfassen.

Der an den ersten Schaftabschnitt 6 anschliessende kreiszylindrische, zweite Schaftabschnitt 7 verfügt über einen im wesentlichen konstanten Kerndurchmesser $d_2$ 17. Der zweite, bis zum Schraubenende 11 reichende Schaftabschnitt 7 weist einen kleineren Kerndurchmesser $d_2$ 17 als der Kerndurchmesser $d_1$ 16 des ersten Schaftschnittes 6 auf. Der Kerndurchmesser $d_2$ 17 des zweiten Schaftabschnittes 7 beträgt bei einem Einsatz der Knochenschraube 1 in der Kieferosteotomie z.B. 6 Millimeter und ist damit um einen Drittel kleiner als der Kerndurchmesser $d_1$ 16 des ersten Schaftabschnittes 6.

Der zweite Schaftabschnitt 7 verfügt über ein Aussengewinde 14, das je nach Länge des zweiten Schaftabschnittes 7 über mehr oder weniger als die fünf dargestellten Windungen 14 mit gleichmässigem Gewindegang 12 verfügen kann. Insbesondere ist der Kerndurchmesser $d_2$ 17 des zweiten Schaftabschnittes 7 zusammen mit den Rippen des Aussengewindes 14 kleiner als der reine Kerndurchmesser $d_1$ 16 des ersten Schaftabschnittes 6, so dass der zweite Schaftabschnitt 7 ohne Berührung von Bohrwänden durch eine für den ersten Schaftabschnitt 6 vorgesehene Bohrung in einem Knochenfragment hindurchführbar ist.

Der Gewindegang 12 des Aussengewindes 14 des zweiten Schaftabschnittes 7 ist gleich dem Gewindegang 10 des ersten Schaftabschnittes 6. Der Übergangsbereich 13 zwischen dem ersten und dem zweiten Schaftabschnitt 6, bzw. 7 weist eine möglichst geringe Höhe auf, die vorteilhafterweise unter der Höhe eines Gewindeganges 10 oder 12 liegt.

Im folgenden wird ein operativer Eingriff mit einem Einsatz von Knochenschrauben 1 gemäss Fig. 1 bei einer Osteotomie zur Unterkieferverlängerung beschrieben. In einer solchen Operation wird der Knochen des Unterkiefers beidseitig gespalten. Anschliessend bringt der Operateur in beide Knochenstücke des Corpus und des Vorderfragmentes jeweils zwei oder drei Bohrungen ein, deren Achsen bei anatomisch richtiger Lagerung der Knochenfragmente miteinander fluchten. Dies führt zu einem zu überbrückenden Hohlraum zwischen den Knochenfragmenten untereinander.

Die einander zugeordneten Bohrungen werden derart ausgestaltet, dass sie unterschiedliche Durchmesser aufweisen. Dabei hat die vordere Bohrung einen grösseren Durchmesser als die hintere Bohrung. Die beiden Bohrungen werden nur mit einem Bohrvorgang mit einem Stufenbohrer, der die verschiedenen Durchmesser hat, hergestellt.

Jede Knochenschraube 1 wird dann mit ihrem zweiten, einen geringeren Kerndurchmesser $d_2$ 17 aufweisenden Schaftabschnitt 7 durch die grössere Bohrung in dem einen Knochenstück, bei der Kieferosteotomie dem Vorderfragment, durchgeführt. Die Schraubenspitze 11 der Knochenschraube 1 dreht sich nun in die im Durchmesser kleinere Öffnung des anderen Knochenstückes, hier dem Corpus, ein, wobei sich bei geeigneter Wahl der Längenverhältnisse der beiden Schaftabschnitte 6 und 7 gleichzeitig der erste, einen grösseren Kerndurchmesser $d_1$ 16 aufweisende Schaftabschnitt 6 in die im Durchmesser grössere Öffnung des Vorderfragmentes eindreht. Das Eindrehen wird in wirkungsvoller Weise durch den Innensechskant 4 des Schraubenkopfes 2 unterstützt, in den der Operateur einen entsprechenden Innensechskant zum sicheren Verdrehen der Knochenschraube 1 einsetzen kann.

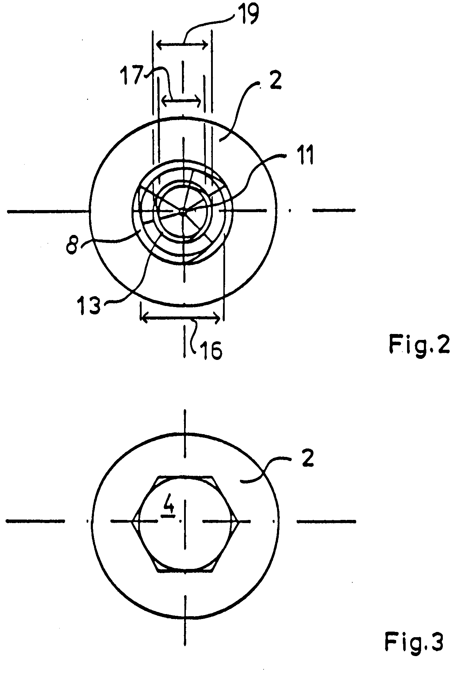

Die Fig. 2 zeigt eine Unteransicht auf die Knochenschraube 1 nach Fig. 1. Es ist deutlich der Unterschied der Kerndurchmesser $d_1$ 16, bzw. $d_2$ 17 der Schaftabschnitte 6, bzw. 7 erkennbar, wobei der Kerndurchmesser $d_2$ 17 des zweiten Schaftabschnittes 7 zusammen mit dem Aussendurchmesser $D_2$ 19 der Rippen des Aussengewindes 14 kleiner als der Kerndurchmesser $d_1$ 16 des ersten Schaftabschnittes 6 ist.

Die Fig. 3 zeigt eine Draufsicht auf die Knochenschraube 1 nach Fig. 1, in welcher der Schraubenkopf 2 mit dem Innensechskant 4 dargestellt ist.

Die Knochenschraube 1 gemäss den Fig. 1 - 3 ist bei einer Vielzahl von Indikationen einsetzbar. Dazu gehört neben der Verkürzungs- und Verlängerungsosteotomie der Einsatz bei der Fixierung von Knochenbruchstücken, z.B. bei Brüchen und Fehlstellungen von Extremitäten.

Eine weitere bevorzugte Ausführungsform der erfindungsgemässen Knochenschraube 1 wird nachstehend anhand der Fig. 4 und 5 beschrieben. Die in Fig. 4 dargestellte Knochenschraube 1 besteht im wesentlichen aus dem Schraubenkopf 2, dem Schaft 5 und der Schraubenspitze 11. Der Schaft 5 verfügt über einen kopfseitigen, glatten Schaftabschnitt 20 und einen spitzenseitigen mit einem Gewinde versehenen Abschnitt 6,7. Letzterer besteht seinerseits aus einem 70 mm langen, kopfseitigen Schaftabschnitt 6 mit einem Kerndurchmesser $d_1$ von 4,65 mm und einem 20 mm langen, spitzenseitigen Schaftabschnitt 7 mit einem Kerndurchmesser $d_2$ von 4,60 mm, wobei die beiden mit einem Gewinde versehenen Schaftabschnitte 6 und 7 durch einen Übergangsbereich 13 mit sich konisch verjüngendem Durchmesser miteinander verbunden sind. Der konische Übergangsbereich 13 weist bei dieser Ausführungsform eine Länge von 5 mm auf und nimmt auf dieser Strecke kontinuierlich vom Kerndurchmesser $d_1$ zum Kerndurchmesser $d_2$, d.h. von 4,65 auf 4,60 mm, ab. Der Übergangsbereich 13 ist in einem Abstand von 20 mm von der Schraubenspitze 11 angebracht. Der Gewindeaussendurchmesser $D_2$ 19 des spitzenseitigen, mit einem Gewinde versehenen zweiten Schaftabschnitts 7 und der Durchmesser $D_s$ 21 des glatten Schaftabschnittes 20 messen beide 5,0 mm. Der spitzenseitige, ein Gewinde tragender Schaftabschnitt 7 besitzt eine Steigung von 1,75 mm.

Die Schraubenspitze 11 der Knochenschraube 1 ist selbstschneidend ausgebildet in Form mehrerer radial über den Umfang verteilten Schneidnuten 22; sie kann auch als Trokarspitze ausgebildet sein. Diese Schneidemittel dienen dazu die Bohrung im Knochen exakt auf den Kerndurchmesser der Knochenschraube 1 auszuweiten und zusätzlich das Gewinde in den Knochen zu schneiden. Aus diesem Grunde kann der Durchmesser der Kernlochbohrung im Knochen geringfügig kleiner sein, ohne dass es bei der Implantation der Knochenschraube 1 zu einer unbeabsichtigten radialen Vorspannung kommt. Wenn für die Kernlochbohrung der genaue Kernlochdurchmesser gebohrt würde, bestünde die Gefahr, dass der Chirurg durch ungenaues Bohren mit einem zu grossen Bohrdurchmesser endet. Die Verwendung eines überdimensionierten Kernlochdurchmessers und die selbstschneidende Schraubenspitze 11 haben den zusätzlichen Vorteil, dass ein sogenannter "Reibahlen-Effekt" erzielt wird. Der "Reibahlen-Effekt" besteht darin, dass die mittels dieser selbstschneidenden Schraubenspitze 11 behandelte Bohrung in der nahen Kortikalis, exakt dem geforderten Ausgangsdurchmesser für die nachfolgende radiale Vorspannung entspricht.

Wie in Fig. 5 angedeutet geschieht das Setzen der erfindungsgemässen Knochenschraube 1 durch ein einmaliges Aufbohren des Röhrenknochens 23,24 mit einem gängigen Bohrer von 4,5 mm Durchmesser, was erfahrungsgemäss einen Bohrlochdurchmesser von 4,55 mm erzeugt. In dieses Bohrloch wird nun die Knochenschraube 1 mit den üblichen hierzu vorgesehen Instrumenten mit ihrem spitzenseitigen Schaftabschnitt 7 des Gewindeteils 6,7 eingeführt. Da der Kerndurchmesser $d_2$ 17 des spitzenseitigen Schaftabschnitts 7 des Gewindeteils 6,7 lediglich 4,60 mm beträgt, wird in der hinteren Kortikalis 23 praktisch keine radiale Vorspannung erzeugt.

Erst wenn der kopfseitige Schaftabschnitt 6 des Gewindeteils 6,7 mit dem Kerndurchmesser $d_1$ 16 über den konischen Übergangsteil 13 in die vordere Kortikalis 24 eingedreht wird, erfolgt eine radiale Vorspannung um den Betrag von 0,05 mm und es ergibt sich schliesslich eine Situation wie in Fig. 5 dargestellt.

**Patentansprüche**

1. Knochenschraube (1) mit einem Schraubenkopf (2), einer Schraubenspitze (11) und einem mindestens teilweise mit einem Gewinde (8,14) versehenen Schaft (5), dadurch gekennzeichnet, dass

A) der mit einem Gewinde versehene Abschnitt (6,7) des Schaftes (5) über einen kopfseitigen, im wesentlichen zylindrischen, ersten Schaftabschnitt (6) mit dem Kerndurchmesser $d_1$ (16) und über einen spitzenseitigen, an den ersten Schaftabschnitt (6) anschliessenden, zweiten Schaftabschnitt (7) mit einem kleineren Kerndurchmesser $d_2$ (17) verfügt und dass

B) das Gewinde (8,14) in den beiden Schaftabschnitten (6,7) die gleiche Steigung (10,12) aufweist.

2. Knochenschraube (1) nach Anspruch 1, dadurch gekennzeichnet, dass zwischen den beiden Schaftabschnitten (6,7) ein Übergangsbereich (13) angeordnet ist.

3. Knochenschraube (1) nach Anspruch 2, dadurch gekennzeichnet, dass sich der Übergangsbereich (13) vom Kerndurchmesser $d_1$ (16) zum Kerndurchmesser $d_2$ (17) hin stetig verjüngt und vorzugsweise konisch ausgebildet ist.

4. Knochenschraube (1) nach Anspruch 2 oder 3, dadurch gekennzeichnet, dass der Übergangsbereich (13) kürzer oder gleich einem Gewindegang (10,12) des Gewindes (8,14) ist.

5. Knochenschraube (1) nach Anspruch 2 oder 3, dadurch gekennzeichnet, dass der Übergangsbereich (13) eine Länge zwischen 3 und 7 mm, vorzugsweise zwischen 4 und 6 mm aufweist.

6. Knochenschraube (1) nach einem der Ansprüche 1 bis 5, dadurch gekennzeichnet, dass das Gewinde (8,14) selbstschneidend ausgebildet ist.

7. Knochenschraube (1) nach einem der Ansprüche 1 bis 6, dadurch gekennzeichnet, dass der Aussendurchmesser (19) der Rippen des Aussengewindes (14) des zweiten Schaftabschnittes (7) kleiner als der Kerndurchmesser (16) des ersten Schaftabschnittes (6) ist.

8. Knochenschraube (1) nach einem der Ansprüche 1 bis 7, dadurch gekennzeichnet, dass der Schaft (5) über einen kopfseitig an seinem mit einem Gewinde versehenen Abschnitt (6,7) anschliessenden, glatten, dritten Schaftabschnitt (20) verfügt.

9. Knochenschraube (1) nach einem der Ansprüche 1 bis 7, dadurch gekennzeichnet, dass das Verhältnis $d_1$-$d_2$/$d_2$ zwischen 0,004 und 0,020, vorzugsweise zwischen 0,008 und 0,012 liegt.

10. Knochenschraube (1) nach Anspruch 8 oder 9, dadurch gekennzeichnet, dass der zweite Schaftabschnitt (7) einen Gewindeaussendurchmesser $D_2$ besitzt, der dem Durchmesser $D_s$ (21) des dritten Schaftabschnittes (20) entspricht.

11. Knochenschraube (1) nach einem der Ansprüche 1 bis 10, dadurch gekennzeichnet, dass die Schraubenspitze (11) selbstschneidend ausgebildet ist, vorzugsweise in Form eines Trokars oder einer oder mehrerer radial über den Umfang verteilten Schneidnuten (22).

12. Knochenschraube (1) nach einem der Ansprüche 1 bis 11, dadurch gekennzeichnet, dass das Verhältnis zwischen Kerndurchmesser $d_2$ - (17) und Aussendurchmesser $D_2$ (19) des zweiten Schaftabschnittes (7) im Bereich zwischen 0,89 und 0,95, vorzugsweise zwischen 0,91 und 0,93 liegt.

13. Knochenschraube (1) nach einem der Ansprüche 2 bis 12, dadurch gekennzeichnet, dass der Übergangsbereich (13) in einem Abstand von 15 - 25 mm, vorzugsweise von 18 - 22 mm von der Schraubenspitze (11) angebracht ist.

14. Knochenschraube (1) nach einem der Ansprüche 1 bis 13, dadurch gekennzeichnet, dass der ersten Schaftabschnitt (6) des Schaftes (5) eine Länge zwischen 60 bis 80 mm, vorzugsweise zwischen 65 bis 75 mm aufweist.

15. Knochenschraube (1) nach einem der Ansprüche 1 bis 14, dadurch gekennzeichnet, dass die Steigung des Gewindeganges (12) des Gewindes (14) des zweiten Schaftabschnittes (7) im Bereich von 1,5 bis 2 mm, vorzugsweise zwischen 1,7 bis 1,8 mm, liegt.

Fig.1

Fig.2

Fig.3

Fig. 4

Fig. 5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 463 753 (R.B.GUSTILO)<br><br>* Zusammenfassung *<br>* Spalte 2, Zeile 30 - Zeile 46; Abbildungen 1-2 *<br><br>--- | 1-3,6-7, 11 | A61B17/58 |
| X | WO-A-9 002 526 (AUSTRALIAN DEFENCE INDUSTRIES)<br><br>* Seite 2, Zeile 17 - Zeile 29; Abbildung 1 *<br>--- | 1-2,4,8, 10 | |
| X | SOVIET INVENTIONS ILLUSTRATED<br>Section PQ, Week 8735, 9. September 1987<br>Derwent Publications Ltd., London, GB;<br>Class P31, AN 87-248872/35<br>& SU-A-1 284 533 (KHARK INSTITUTE OF ORTHOPAEDICS AND TRAUMATOLOGY) 23. Januar 1987<br>* Zusammenfassung *<br>--- | 1-2,7-8 | |
| D,A | EP-A-0 369 266 (SYNTHES)<br>* Ansprüche; Abbildungen *<br><br>----- | 1-15 | |
| | | | **RECHERCHIERTE SACHGEBIETE (Int. Cl.5)**<br><br>A61B<br>F16B |

Der vorliegende Recherchenbericht wurde für alle Patentansprüche erstellt

| Recherchenort | Abschlußdatum der Recherche | Prüfer |
|---|---|---|
| DEN HAAG | 20 FEBRUAR 1992 | NICE P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument

EPO FORM 1503 03.82 (P0403)